# EUROPEAN PATENT SPECIFICATION

(11) **EP 3 777 955 B1**
(45) Date of publication and mention of the grant of the patent: **26.07.2023**
(21) Application number: 19776888.0
(22) Date of filing: 25.03.2019
(51) Int. Cl.: B29C 39/24, A61M 37/00, B29C 33/38, B29C 39/42, B29C 39/44, B29C 45/14, B29C 45/26, B29C 33/40, B29L 31/00, B29C 41/04

(54) **MICRONEEDLE ARRAY MANUFACTURING METHOD**
MIKRONADELANORDNUNGHERSTELLUNGSVERFAHREN
PROCÉDÉ DE FABRICATION D'UN RÉSEAU DE MICRO-AIGUILLES

(30) Priority: 30.03.2018 JP 2018068102
(43) Date of publication of application: 17.02.2021
(73) Proprietor: FUJIFILM Corporation, Tokyo 106-8620 (JP)
(72) Inventor: TAKANO, Ikuo, Odawara-shi, Kanagawa 250-0001 (JP)
(74) Representative: Klunker IP Patentanwälte PartG mbB
(86) International application number: PCT/JP2019/012412
(87) International publication number: WO 2019/188935

(56) References cited:
- EP-A1- 3 006 078
- EP-A1- 3 235 537
- EP-A1- 3 269 416
- WO-A1-2008/131383
- WO-A1-2014/077242
- WO-A1-2017/056894
- WO-A1-2017/195545
- WO-A1-2017/208962
- JP-A- 2008 142 183
- JP-A- 2012 200 572
- JP-A- 2016 112 169

## Description

### BACKGROUND OF THE INVENTION

### 1. Field of the Invention

The present invention relates to a method for manufacturing a microneedle array, and particularly to a method for manufacturing a microneedle array in which a drug solution is ejected from a drug solution ejection nozzle toward a needle-like recessed portion of a mold.

### 2. Description of the Related Art

In recent years, a microneedle array (percutaneous absorption sheet) formed with needle-like protrusions (also referred to as small needles or microneedles) containing a drug has been used to deliver the drug into the skin. In general, by pressing the microneedle array against the skin to insert the needle-like protrusions into the skin, the drug of the needle-like protrusions is delivered into the skin.

As a method of manufacturing a microneedle array, a method has been known in which a mold formed with a needle-like recessed portion (also referred to as a needle hole portion) that is an inverted shape of a needle-like protrusion is used, the needle hole portion is filled with a solution (also referred to as a drug solution) containing a drug and is dried, and thereafter a solution containing a needle (also referred to as needle) raw material is applied and dried to form a microneedle.

In particular, in a step of filling the needle-like recessed portion with the drug solution, since the filling amount thereof is related to the drug dose, it is necessary to reliably fill a mold sheet with a very small amount of the drug solution in a constant amount with high accuracy.

Hitherto, several methods have been proposed for filling a drug solution (refer to WO2014/077242A, JP2010-069253A, JP2013-162982A, and JP2016-112169A).

EP3269416A1 discloses a process for producing sheet for percutaneous absorption. The method includes a drug solution filling step of filling needle-like recessed portions of a mold having the needle-like recessed portions with a drug solution that is a polymer solution containing a drug, a drug solution drying step, a polymer layer forming solution supply step, and a polymer layer forming solution drying step to form a polymer layer. EP3006078A1 discloses a needle body manufacturing method including a first step of supplying a needle forming solution onto an intaglio plate having a recess that has a shape corresponding to the needle, a second step of disposing an air-permeable film on the needle forming solution and compressing the needle forming solution from above the film to move the needle forming solution into the recess, and a third step of removing liquid components from the needle forming solution inside the recess to solidify the needle forming solution, and forming the needle.

### SUMMARY OF THE INVENTION

However, the filling methods described in WO2014/077242A and JP2010-069253Ahave a problem that it is not possible to accurately fill the needle-like recessed portion of the mold with the drug solution.

In addition, in JP2013-162982A and JP2016-112169A, although the filling methods in which liquid droplets are ejected to the needle-like recessed portion of the mold are used, in a case where filling is performed at a high speed in order to improve productivity, there is a problem that the landing position of the liquid droplet and the position of the needle-like recessed portion of the mold deviate from each other.

In a case where the drug solution deviates from the center of the needle-like recessed portion of the mold or the drug solution adheres to the wall surface of the needle-like recessed portion and thus the drug solution cannot close the needle-like recessed portion, the drug solution cannot fill the distal end of the needle-like recessed portion, and there is a problem that the manufacturing yield of a microneedle array is reduced.

The present invention has been made taking the above circumstances into consideration, and an object thereof is to provide a method for manufacturing a microneedle array in which the distal end of a needle-like recessed portion is reliably filled with a drug solution.

In one aspect of the present invention, there is provided a method for manufacturing a microneedle array, according to claim 1 of the appended claims.

According to the aspect, it is possible to cause the ejected drug solution to land on the needle-like recessed portion and fill the distal end of the needle-like recessed portion. The position of the needle-like recessed portion and the position of the drug solution ejection nozzle may coincide with each other so that the drug solution ejected from the drug solution ejection nozzle toward the needle-like recessed portion lands on the needle-like recessed portion, and the positions of the two do not need to strictly coincide with each other.

By causing the opening diameter to be 600 µm or more, the ejected drug solution can be easily landed on the needle-like recessed portion, by causing the angle formed with the flat portion of the mold to be 30.0 degrees or more, the landed drug solution can be easily collected or flow into the center of the needle-like recessed portion, and by causing the ejection amount of the drug solution to be 30 nL or more, it is possible to easily block the needle-like recessed portion. Accordingly, the ejected drug solution can be caused to land on the needle-like recessed portion and fill the distal end of the needle-like recessed portion.

It is preferable that the opening diameter of the needle-like recessed portion is 1200 µm or less. Accordingly, the ejected drug solution can be caused to land on the needle-like recessed portion.

It is preferable that the angle of the needle-like recessed portion is 90.0 degrees or less. Accordingly, the landed drug solution can be easily collected or flow into the center of the needle-like recessed portion.

It is preferable that the ejection amount is 150 nL or less. Accordingly, the needle-like recessed portion can be blocked by the ejected drug solution.

It is preferable that a first edge portion of an opening portion of the needle-like recessed portion is chamfered, and a first radius of curvature of a chamfer of the first edge portion is 30 µm or more. Accordingly, it is possible to prevent the drug solution landed on the needle-like recessed portion from being pinned against the first edge portion, and to easily cause the drug solution to be collected or flow into the center of the needle-like recessed portion so as to fill the distal end of the needle-like recessed portion.

It is preferable that the first radius of curvature is 300 µm or less. Accordingly, it is possible to prevent the drug solution landed on the needle-like recessed portion from being pinned against the first edge portion, and to easily cause the drug solution to be collected to flow into the center of the needle-like recessed portion so as to fill the distal end of the needle-like recessed portion.

It is preferable that the needle-like recessed portion comprises a cup portion provided on the front surface of the mold, and a distal end recessed portion which is connected to the cup portion and has a tapered shape in a depth direction of the mold, a second edge portion between the cup portion and the distal end recessed portion is chamfered, and a second radius of curvature of a chamfer of the second edge portion is 30 µm or more. Accordingly, it is possible to prevent the drug solution that has landed on the needle-like recessed portion from being pinned against the second edge portion, and to easily cause the drug solution to be collected or flow into the center of the needle-like recessed portion so as to fill the distal end of the needle-like recessed portion.

It is preferable that the second radius of curvature is 300 µm or less. Accordingly, it is possible to prevent the drug solution ejected to the needle-like recessed portion from being pinned against the second edge portion, and to easily cause the drug solution to be collected or flow into the center of the needle-like recessed portion so as to fill the distal end of the needle-like recessed portion.

It is preferable that a flight velocity of the drug solution ejected from the drug solution ejection nozzle is 0.2 m/s or more. Accordingly, it is possible to easily cause the ejected drug solution to be collected or flow into the center of the needle-like recessed portion.

It is preferable that the flight velocity of the drug solution ejected from the drug solution ejection nozzle is 1.0 m/s or less. Accordingly, it is possible to easily cause the ejected drug solution to be collected or flow into the center of the needle-like recessed portion.

It is preferable that an imaging step of imaging a plurality of positions of the mold is comprised, in the positioning adjustment step, the position of the needle-like recessed portion is calculated based on the plurality of positions imaged, and in the relative movement step, the position of the needle-like recessed portion and a landing position of the drug solution ejected from the drug solution ejection nozzle are caused to coincident with each other based on a result of the calculation. Accordingly, the ejected drug solution can be caused to appropriately land on the needle-like recessed portion.

It is preferable that the mold has a plurality of the needle-like recessed portions, and in the suction step, the rear surface of the mold is suctioned after the drug solution is ejected into all the needle-like recessed portions of the plurality of needle-like recessed portions. Accordingly, the drug solution that has landed on the needle-like recessed portion can be reliably filled.

It is preferable that the mold has gas permeability. Accordingly, appropriate suctioning is possible, and the drug solution that has landed on the needle-like recessed portion can be reliably filled.

It is preferable that a drying step of drying the drug solution with which the needle-like recessed portion is filled is further comprised. Accordingly, the microneedle array containing the drug solution can be manufactured.

It is preferable that the drug solution contains at least one of a drug stock solution, a sugar, or an additive. Accordingly, the microneedle array can be manufactured according to the application.

According to the aspect of the present invention, the distal end of the needle-like recessed portion can be reliably filled with the drug solution.

### BRIEF DESCRIPTION OF THE DRAWINGS

Fig. 1 is a perspective view illustrating an example of a percutaneous absorption sheet.
Fig. 2 is a perspective view illustrating an example of a mold.
Fig. 3 is a partially enlarged view of a section 3-3 in Fig. 2.
Fig. 4 is a process diagram illustrating a method for producing a mold by injection molding.
Fig. 5 is a process diagram illustrating the method for producing a mold by injection molding.
Fig. 6 is a process diagram illustrating the method for producing a mold by injection molding.
Fig. 7 is a process diagram illustrating the method for producing a mold by injection molding.
Fig. 8 is a process diagram illustrating the method for producing a mold by injection molding.
Fig. 9 is a process diagram illustrating the method for producing a mold by injection molding.
Fig. 10 is a process diagram illustrating the method for producing a mold by injection molding.
Fig. 11 is a process diagram illustrating the method for producing a mold by injection molding.
Fig. 12 is a process diagram illustrating the method for producing a mold by injection molding.
Fig. 13 is a perspective view of a transporting jig on which a mold is mounted.
Fig. 14 is a flowchart showing each step of a method for manufacturing a percutaneous absorption sheet.
Fig. 15 is a schematic configuration diagram of a drug solution filling apparatus used in a drug solution filling step.
Fig. 16 is a block diagram illustrating an electrical configuration of the drug solution filling apparatus.
Fig. 17 is a flowchart showing each step included in the drug solution filling step.
Fig. 18 is a cross-sectional view for describing the shape of a needle-like recessed portion according to an example.
Fig. 19 is a table showing each level and evaluation results thereof.

### DESCRIPTION OF THE PREFERRED EMBODIMENTS

Hereinafter, preferred embodiments of the present invention will be described with reference to the accompanying drawings. The present invention is described by the following preferred embodiments.

Here, in the figures, like elements having similar functions are denoted by like reference numerals. In addition, in this specification, in a case where a numerical value range is expressed using "to", the numerical value range includes the numerical values of the upper limit and the lower limit indicated by "to".

### <Configuration of Percutaneous Absorption Sheet>

First, an example of a microneedle array (percutaneous absorption sheet) manufactured by a method for manufacturing a microneedle array in the present embodiment will be described.

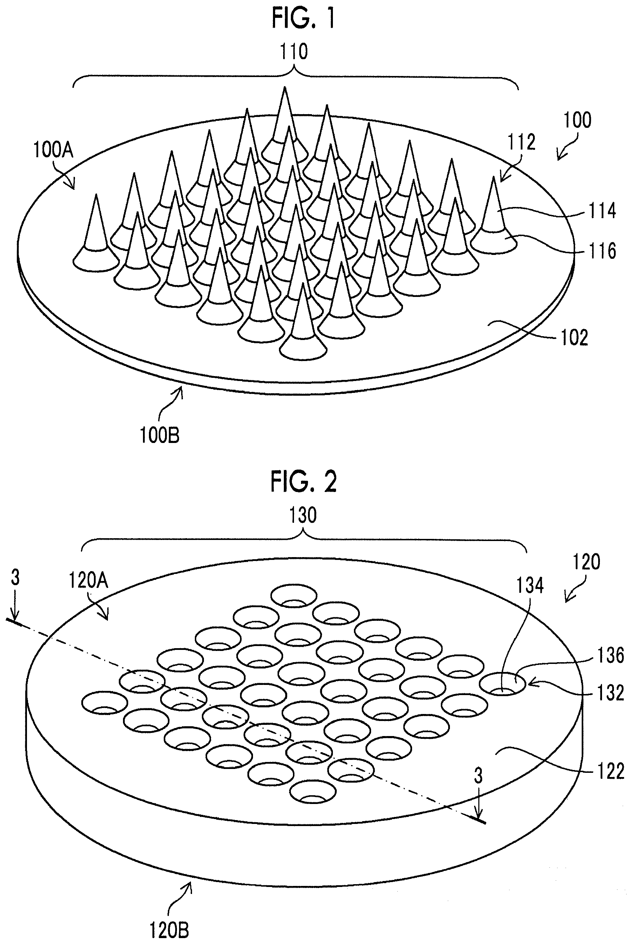

Fig. 1 is a perspective view illustrating an example of a percutaneous absorption sheet 100. The percutaneous absorption sheet 100 has a front surface 100A and a rear surface 100B, and is constituted by a sheet-like sheet portion 102 and a protruding pattern 110.

"Sheet-like" means a thin flat shape as a whole with respect to the two opposing front and rear surfaces 100A and 100B having a large area, and the front and rear surfaces 100A and 100B do not need to be completely flat. Although the sheet portion 102 illustrated in Fig. 1 is circular in a plan view, the sheet portion 102 may be rectangular, polygonal, elliptical, or the like.

The protruding pattern 110 has a plurality of needle-like protrusions 112. The needle-like protrusions 112 are provided on the front surface 100A. The needle-like protrusion 112 includes a needle portion 114, and a frustum portion 116 that connects the needle portion 114 to the sheet portion 102.

A plurality of the frustum portions 116 are formed on the front surface 100A of the percutaneous absorption sheet 100. The frustum portion 116 has two bottom surfaces and has a three-dimensional structure surrounded by a conical surface. The bottom surface (lower bottom surface) of the two bottom surfaces of the frustum portion 116 having a large area is connected to the sheet portion 102. The bottom surface (upper bottom surface) of the two bottom surfaces of the frustum portion 116 having a small area is connected to the needle portion 114. That is, of the two bottom surfaces of the frustum portion 116, the area of the bottom surface in a direction away from the sheet portion 102 is small.

The needle portion 114 has a bottom surface with a large area and a shape having a narrowest area at the distal end apart from the bottom surface. Since the bottom surface of the needle portion 114 having a large area is connected to the upper bottom surface of the frustum portion 116, the needle portion 114 has a tapered shape in a direction away from the frustum portion 116. Therefore, the needle-like protrusion 112 constituted by the needle portion 114 and the frustum portion 116 has a tapered shape as a whole from the sheet portion 102 toward the distal end. A plurality of, for example, 4 to 2500 needle-like protrusions 112 are provided on the sheet portion 102. However, the number thereof is not limited thereto.

In Fig. 1, the frustum portion 116 has a truncated cone shape, and the needle portion 114 has a cone shape. Depending on the degree of insertion of the needle portion 114 into the skin, the shape of the distal end of the needle portion 114 can be appropriately changed to a curved surface having a radius of curvature of 0.01 µm or more and 50 µm or less, a flat surface, or the like.

### <Configuration of Mold>

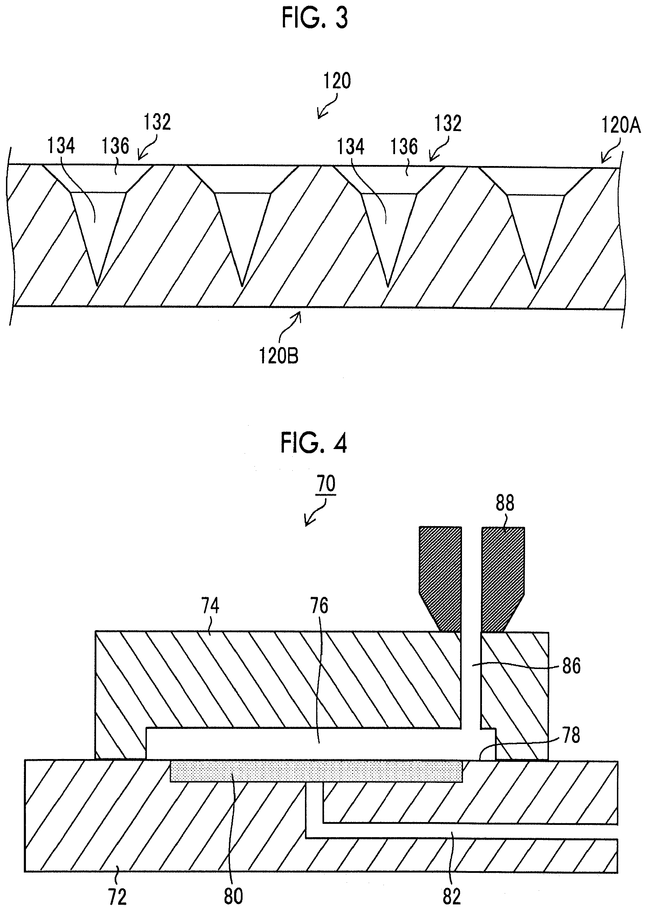

Fig. 2 is a perspective view illustrating an example of a mold 120 (mold for manufacturing a percutaneous absorption sheet) for manufacturing the percutaneous absorption sheet 100. In addition, Fig. 3 is a partially enlarged view of a section 3-3 in Fig. 2. The mold 120 has a front surface 120A and a rear surface 120B, and is constituted by a flat portion 122 and a recessed pattern 130.

The flat portion 122 has a flat shape corresponding to the sheet portion 102 of the percutaneous absorption sheet 100. The recessed pattern 130 is constituted by a plurality of needle-like recessed portions 132. The needle-like recessed portion 132 has a shape corresponding to the needle-like protrusion 112 of the percutaneous absorption sheet 100, and is constituted by a distal end recessed portion 134 corresponding to the needle portion 114 and a cup portion 136 corresponding to the frustum portion 116.

The distal end recessed portion 134 has a tapered shape in a depth direction of the mold 120. The distal end recessed portion 134 can have a diameter of 150 µm to 500 µm and a height of 150 µm to 2000 µm. The cup portion 136 is provided with an opening in the front surface 120A of the mold 120, has a shape that narrows in the depth direction of the mold 120, and is connected to the distal end recessed portion 134 at the narrowest portion. The cup portion 136 can have a diameter of 500 µm to 1000 µm and a height of 100 µm to 500 µm.

The shape of the needle-like recessed portion 132 is not limited to this example. A rocket shape provided with an intermediate recessed portion having a constant width in the depth direction, such as a cylinder, a quadrangular prism, or a polygonal column, between the distal end recessed portion 134 and the cup portion 136 may be applied. In addition, a through-hole that reaches the rear surface 120B and penetrates the mold 120 may be formed at the distal end of the tapered shape. The arrangement, pitch, number, and the like of the needle-like recessed portions 132 are determined based on the arrangement, pitch, number, and the like of the needle-like protrusions 112 necessary for the percutaneous absorption sheet 100.

### <Method for Producing Mold>

A method for producing a mold by injection molding will be described with reference to process diagrams of Figs. 4 to 12.

As illustrated in Fig. 4, a mold 70 including a first mold 72 and a second mold 74 is prepared. By clamping the first mold 72 and the second mold 74, a cavity 76 is formed inside the mold 70. The cavity 76 means a space filled with a resin.

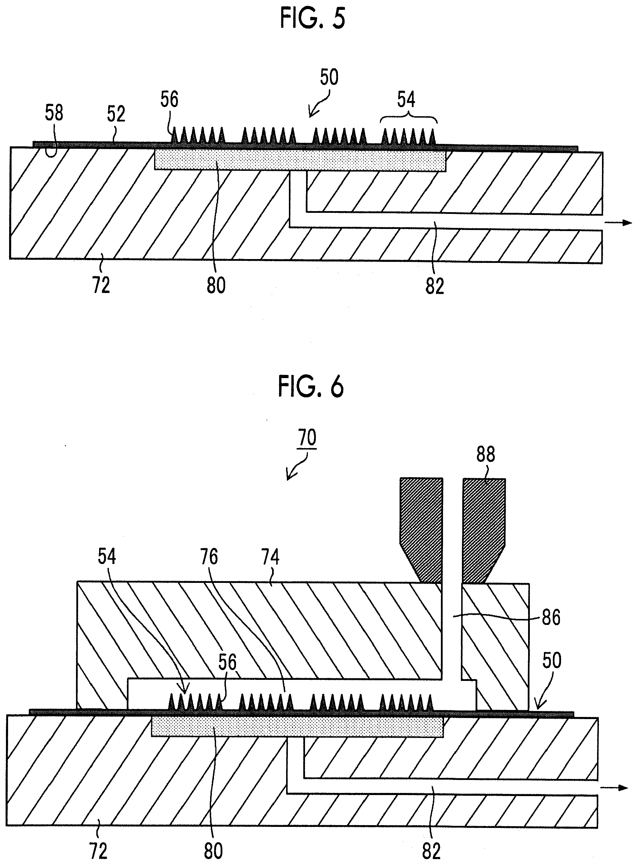

In addition, as illustrated in Fig. 5, an electroform 50 is prepared. On a first surface 52 of the electroform 50, a protruding pattern 54 which is an inverted shape of a mold to be produced is formed. The protruding pattern 54 is a state in which a plurality of needle-like protrusions 56 are arranged in an array. The needle-like protrusions 56 are produced according to the shape of the mold to be produced.

Since the electroform 50 is fixed to the first mold 72, the side to which the electroform 50 is fixed is formed of the flat surface 78. The first mold 72 comprises an adsorption plate 80 on the flat surface 78 as a device for fixing the electroform 50. The first mold 72 comprises a suction pipe 82 in which gas communicates with the adsorption plate 80. The suction pipe 82 is connected to a vacuum pump (not illustrated). By driving the vacuum pump, air can be suctioned from the front surface of the adsorption plate 80. For example, the adsorption plate 80 is formed of a porous member. Examples of the porous member include a metal sintered body, a resin, and a ceramic.

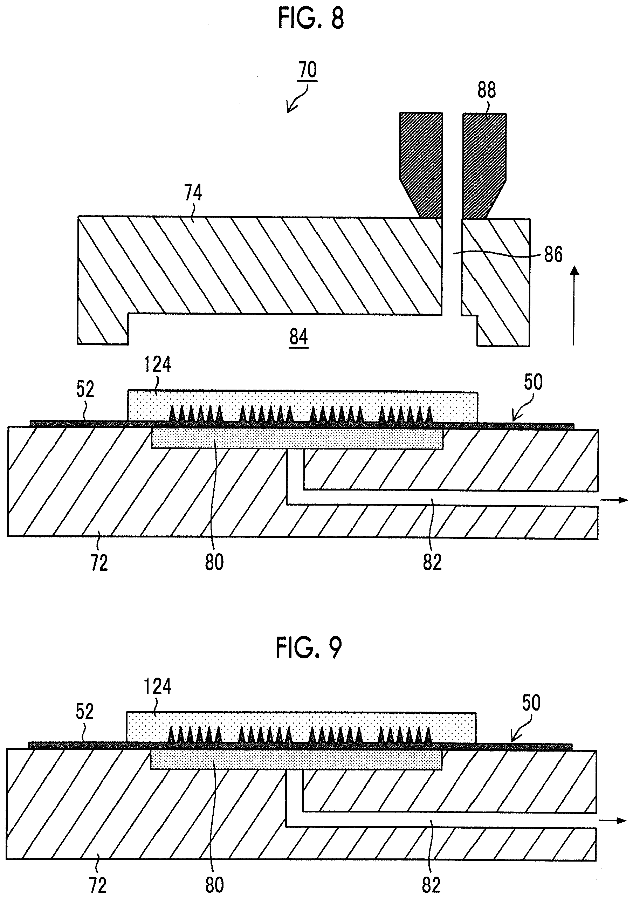

A depression 84 is formed on the cavity 76 side of the second mold 74. In the present embodiment, the cavity 76 is formed by the flat surface 78 of the first mold 72 and the depression 84 (see Fig. 8) of the second mold 74. By configuring the first mold 72 and the second mold 74 as described above, releasing of the mold is facilitated as described later.

A gate 86 that communicates with the cavity 76 is formed in the second mold 74. The gate 86 serves as an injection port for a resin into the cavity 76 of the mold 70. The gate 86 communicates with an injection molding machine 88 that supplies the resin into the mold 70.

As illustrated in Fig. 5, the first mold 72 and the second mold 74 are opened, and the electroform 50 having the protruding patterns 54 is placed on the first mold 72. By suctioning air using the vacuum pump via the suction pipe 82, the second surface 58 of the electroform 50 is vacuum-adsorbed onto the adsorption plate 80.

In the present embodiment, the case where the electroform 50 is fixed to the first mold 72 by vacuum adsorption is illustrated, but the present embodiment is not limited thereto. For example, instead of the adsorption plate 80, a magnet may be provided in the first mold 72 to fix the electroform 50 to the first mold 72 by utilizing the magnetic force. Therefore, it is preferable to fix the electroform 50 to the first mold 72 by at least one of vacuum adsorption or magnetic force.

As illustrated in Fig. 6, in order to form the cavity 76, the first mold 72 and the second mold 74 are clamped. During the clamping, the electroform 50 is clamped by the first mold 72 and the second mold 74.

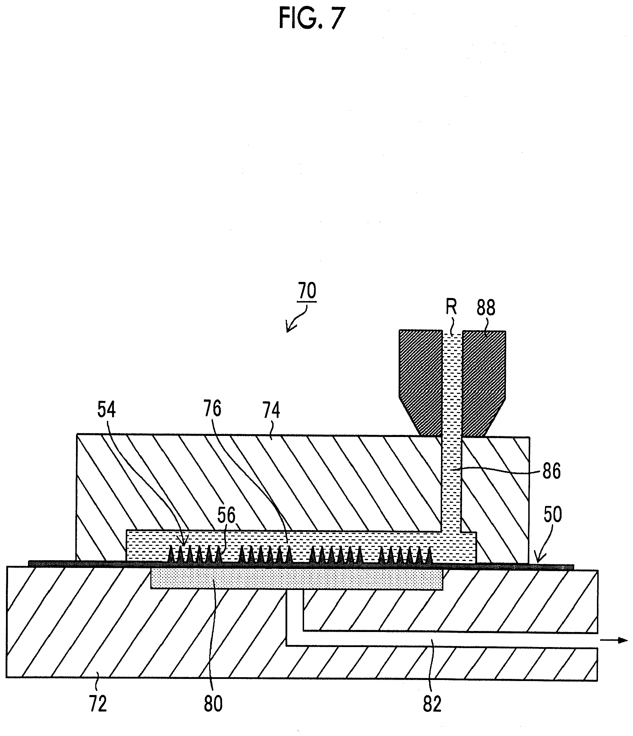

As illustrated in Fig. 7, the resin R is supplied from the injection molding machine 88 to the cavity 76 via the gate 86. The resin R fills the cavity 76 while passing through between the protruding patterns 54 of the electroform 50. As the resin R, a thermosetting resin or a silicone resin is preferably used, and particularly, a silicone resin is preferably used. In a case where the cavity 76 of the mold 70 is filled with the resin R, the resin R is then heated and the resin R is cured.

As illustrated in Fig. 8, in order to release the cured resin R from the electroform 50, the first mold 72 and the second mold 74 clamped are opened. During the opening, the first mold 72 and the second mold 74 are moved away from each other. As illustrated in Fig. 8, the second mold 74 has the depression 84 for forming the cavity 76. The cured resin R is a mold 124 on which a plurality of recessed patterns 130 before releasing are formed.

As illustrated in Fig. 9, the first mold 72 is separated from the second mold 74 and is moved to a stage for releasing the mold 124 from the electroform 50. In the present embodiment, since the second mold 74 having the depression 84 is separated from the mold 124, the mold 124 excluding the surface being in contact with the electroform 50 fixed to the first mold 72 is exposed. Therefore, in a case where the mold 124 is released from the electroform 50, it is possible to easily release the mold 124 using the exposed surface of the mold 124.

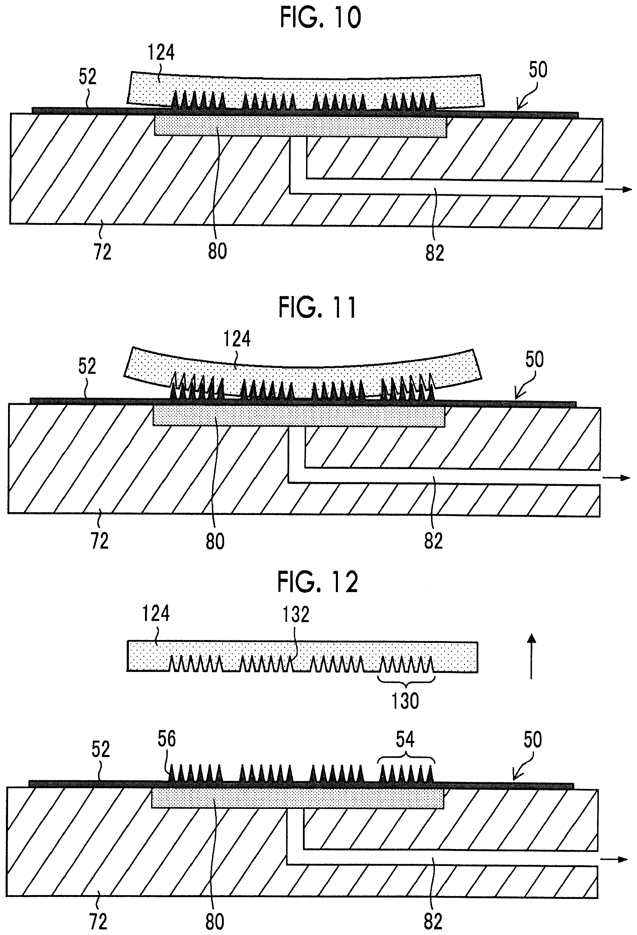

As illustrated in Fig. 10, the peripheral portion of the mold 124 is first separated from the electroform 50. The peripheral portion of the mold 124 may include at least two opposing sides in a case where the mold 124 is viewed in a plan view, and may include all of the four sides. The peripheral portion means a region from the outer periphery of the mold 124 to the recessed pattern 130.

As illustrated in Fig. 11, the peripheral portion of the mold 124 is gradually separated from the electroform 50. In a case where the mold 124 is made of a silicone resin, since the mold 124 has elasticity, the mold 124 enters a stretched state (elastically deforms) as the peripheral portion of the mold 124 is gradually separated. As the peripheral portion of the mold 124 is further separated from the electroform 50, the elastically deformed mold 124 tries to return to its original shape, so that the mold 124 contracts. By using the contraction force of the mold 124, the mold 124 is released from the electroform 50. By using the contraction force of the mold 124 as the releasing force, no excessive force is applied between the mold 124 and the protruding patterns 54 of the electroform 50, so that it is possible to suppress failure in releasing.

As illustrated in Fig. 12, finally, the mold 124 and the protruding patterns 54 of the electroform 50 are completely released from each other, and the mold 124 having the recessed patterns 130 is produced. The mold 124 is in a state in which a plurality of the molds 120 illustrated in Fig. 2 are connected.

In a case where the mold 124 is repeatedly produced from the electroform 50, the protruding patterns 54 are gradually damaged, and after use about 1000 to 10,000 times, it is necessary to replace the electroform 50 with a new electroform 50. In the present embodiment, by stopping the driving of the vacuum pump (not illustrated) and reducing the adsorption force of the adsorption plate 80, the electroform 50 can be replaced within a short period of time.

As a method of separating the peripheral portion of the mold 124 from the electroform 50, there is a method of suctioning the peripheral portion of the mold 124 in the exposed surface opposite to the surface on which the recessed patterns 130 are formed with suctioning means and separating the suctioning means from the electroform 50 while suctioning the peripheral portion.

It is desirable that the mold 124 (mold 120) produced in this manner has excellent gas permeability.

### <Method for Manufacturing Percutaneous Absorption Sheet>

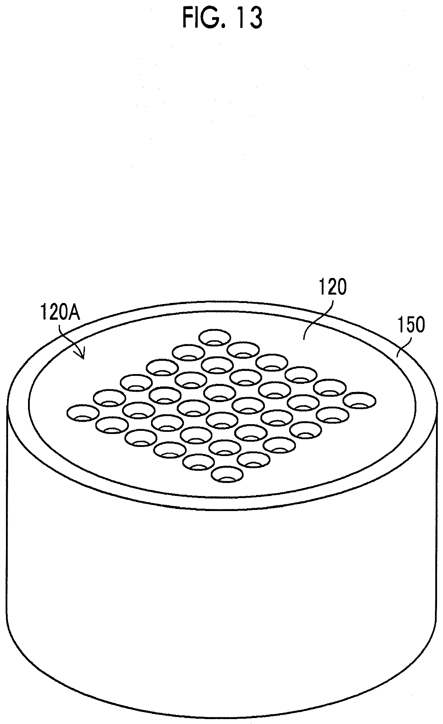

Fig. 13 is a perspective view of a transporting jig 150 on which the mold 120 is mounted. In the manufacturing of the percutaneous absorption sheet, the mold 120 is mounted on the transporting jig 150 and handled. The transporting jig 150 is made of a plastic such as polypropylene. The transporting jig 150 supports the mold 120 in a state in which the front surface 120A of the mold 120 faces upward in a Z direction, which is a vertical direction, and the sheet portion 102 of the mold 120 is parallel to an XY plane, which is a horizontal plane.

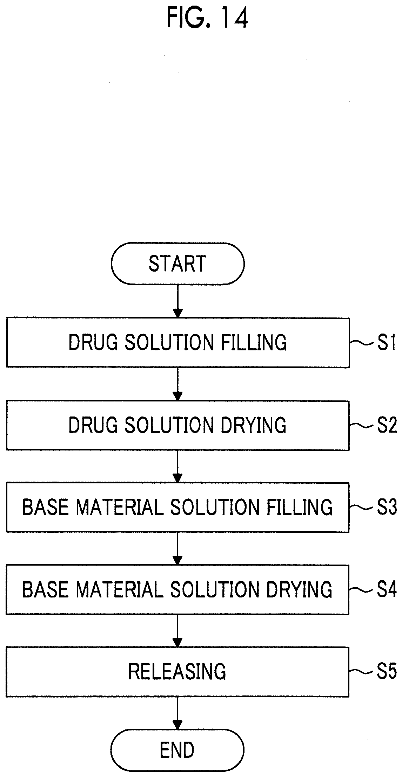

Fig. 14 is a flowchart showing each step of a method for manufacturing the percutaneous absorption sheet 100. The method for manufacturing the percutaneous absorption sheet 100 includes a drug solution filling step (step S1) of filling the needle-like recessed portion 132 of the mold 120 with a drug solution, a drug solution drying step (step S2) of drying the filled drug solution, a base material solution filling step (step S3) of filling the needle-like recessed portion 132 with a base material solution, a base material solution drying step (step S4) of drying the filled base material solution, and a releasing step (step S5) of releasing the formed percutaneous absorption sheet 100 from the mold 120.

### [Drug Solution Filling Step (Step S1)]

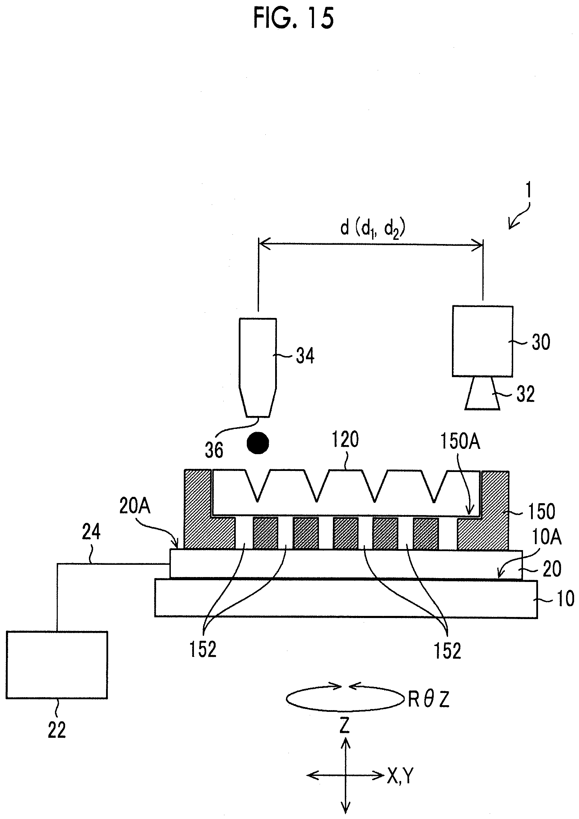

In the drug solution filling step, a liquid droplet of the drug solution is ejected from a nozzle 36 (see Fig. 15) of a drug solution ejection head 34 toward the needle-like recessed portion 132 of the mold 120, and the mold 120 is suctioned by a suction pump 22 (see Fig. 15). Details of the drug solution filling step will be described later.

### [Drug Solution Drying Step (Step S2)]

In the drug solution drying step, for example, drying is performed by blowing air to the drug solution filling the needle-like recessed portion 132. The environment around the mold 120 may be reduced in pressure.

### [Base Material Solution Filling Step (Step S3)]

In the base material solution filling step, the needle-like recessed portion 132 is filled with the base material solution. The base material solution is a drug-free polymer solution, and as water-soluble polymer substance forming the polymer solution, it is preferable to use a water-soluble polymer substance such as chondroitin sulfate, hydroxyethyl starch, or dextran.

Examples of a method for filling the needle-like recessed portion 132 with the base material solution include application using a spin coater.

### [Base Material Solution Drying Step (Step S4)]

In the base material solution drying step, as in the drug solution drying step, drying is performed by blowing air to the base material solution filling the needle-like recessed portion 132.

### [Releasing Step (Step S5)]

In the releasing step, the sheet (percutaneous absorption sheet 100) formed by drying the drug solution and the base material solution is released from the mold 120.

### <Drug Solution Filling Apparatus>

Fig. 15 is a schematic configuration diagram of a drug solution filling apparatus 1 (an example of an apparatus for manufacturing a percutaneous absorption sheet) used in the drug solution filling step. The drug solution filling apparatus 1 includes an XYZ stage 10, an adsorption plate 20, the suction pump 22, a camera 30, the drug solution ejection head 34, and the like.

The XYZ stage 10 (an example of a positioning unit) has a placement surface 10A parallel to an XY plane. The XYZ stage 10 is provided so as to be movable by a motor (not illustrated) in an X direction and a Y direction orthogonal to the X direction, which are two directions parallel to the XY plane. Furthermore, the XYZ stage 10 is provided so as to be movable also in a Z direction and an RθZ direction, which is a rotation direction with the direction parallel to the Z direction as the rotation axis.

The adsorption plate 20 is fixed to the placement surface 10A of the XYZ stage 10. The adsorption plate 20 has a placement surface 20A parallel to the XY plane. The placement surface 20A is provided with a plurality of adsorption holes (not illustrated). The adsorption plate 20 may be made of a porous member.

The suction pump 22 is connected to the adsorption plate 20 via a suction pipe 24. By driving the suction pump 22, air can be suctioned from the plurality of adsorption holes (not illustrated) of the placement surface 20A of the adsorption plate 20.

The transporting jig 150 is placed on the placement surface 20A of the adsorption plate 20. In the transporting jig 150, the mold 120 is mounted on a placement surface 150A. Accordingly, the mold 120 can move in each direction as the XYZ stage 10 moves in the X direction, the Y direction, the Z direction, and the RθZ direction.

A plurality of adsorption holes 152 pass through the placement surface 150A of the transporting jig 150. By driving the suction pump 22, the rear surface 120B of the mold 120 is suctioned via the plurality of adsorption holes (not illustrated) of the placement surface 20A of the adsorption plate 20 and the plurality of adsorption holes 152 of the transporting jig 150.

The camera 30 comprises, in addition to an imaging lens 32, an imaging element (not illustrated), an analog-to-digital converter, and an image processing circuit.

The imaging lens 32 is a lens group comprising a zoom lens, a focus lens, and the like, and causes incidence ray from a subject to be incident onto the imaging element.

The imaging element is a charge coupled device (CCD) type imaging element or a complementary metal oxide semiconductor (CMOS) type imaging element in which a large number of light-receiving elements are two-dimensionally arranged on an imaging surface (not illustrated). The imaging element is disposed in a rear stage of an optical path of the incidence ray of the imaging lens 32.

The imaging lens 32 forms an image of the incidence ray on an imaging surface of the imaging element. The imaging element outputs an analog imaging signal corresponding to the amount of received light. This imaging signal is converted into a digital signal by the analog-to-digital converter, and then generated into an image signal by the image processing circuit.

The camera 30 is disposed above the XYZ stage 10 in the Z direction, and the imaging lens 32 is directed downward in the Z direction. Accordingly, the camera 30 can image the mold 120 placed on the XYZ stage 10.

The drug solution ejection head 34 is disposed at a position above the XYZ stage 10 in the Z direction and separated from the camera 30 by a distance d on the XY plane including a distance d₁ in the X direction and a distance d₂ in the Y direction. The drug solution ejection head 34 includes the nozzle 36 (an example of a drug solution ejection nozzle) that ejects liquid droplets of the drug solution in a first direction. Here, the nozzle 36 is directed downward in the Z direction, and the first direction is a downward direction in the Z direction. The drug solution ejection head 34 illustrated in Fig. 15 includes one nozzle 36, but may include a plurality of nozzles 36.

As the drug solution ejection head 34, for example, an inkjet head such as a solenoid type ink jet head or a piezoelectric ink jet head can be used. The amount of one liquid droplet ejected from the nozzle 36 is about 1 to 150 nL.

The drug solution ejected from the nozzle 36 flies downward in the Z direction and lands on an object (in this case, the mold 120). Therefore, the position of the nozzle 36 on the XY plane and the position on the XY plane where the drug solution lands are the same.

The drug solution contains a drug stock solution, a sugar, an additive, and the like as the drug. Moreover, the drug solution contains water, ethanol, or the like as a solvent.

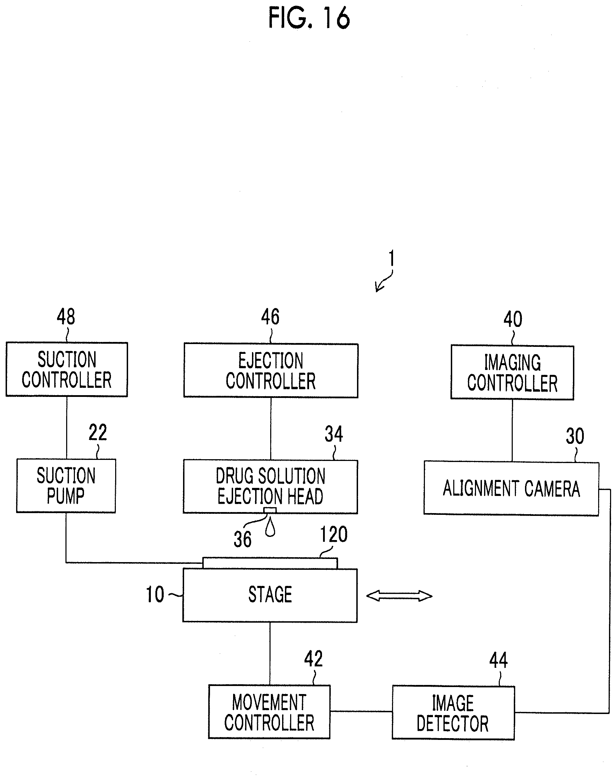

Fig. 16 is a block diagram illustrating an electrical configuration of the drug solution filling apparatus 1. The drug solution filling apparatus 1 comprises an imaging controller 40, a movement controller 42, an image detector 44, an ejection controller 46, a suction controller 48, and the like.

The imaging controller 40 causes the camera 30 to take an image.

The movement controller 42 controls a relative movement between the mold 120 placed on the XYZ stage 10 and the drug solution ejection head 34. Here, the mold 120 is moved by driving the XYZ stage 10, but the drug solution ejection head 34 may be moved, or both the mold 120 and the drug solution ejection head 34 may be moved.

The image detector 44 detects the position of the mold 120 based on the image of the mold 120 imaged by the camera 30. In the present embodiment, the position of the needle-like recessed portion 132 is detected by recognizing the needle-like recessed portion 132 from the image of the mold 120.

The ejection controller 46 controls the timing of ejecting the drug solution from the nozzle 36, and the liquid droplet amount of the drug solution to be ejected, by controlling the drug solution ejection head 34.

The suction controller 48 controls the presence or absence of suction by the suction pump 22.

### <Drug Solution Filling Step>

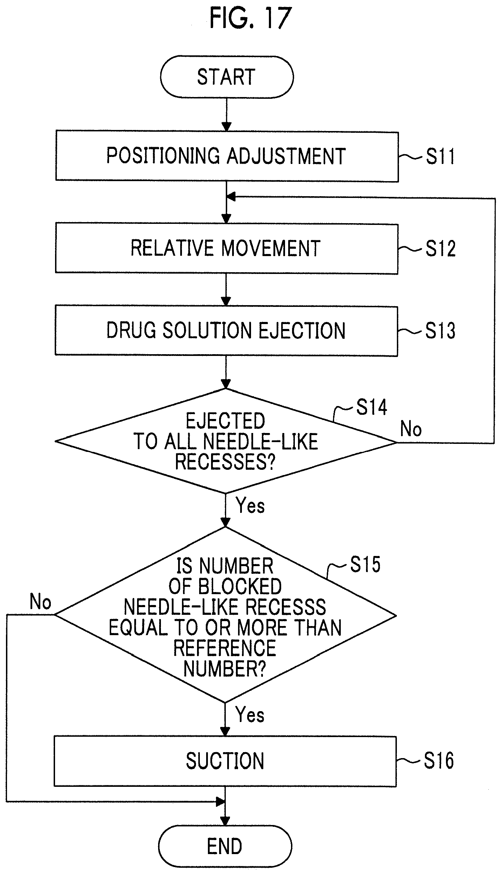

Fig. 17 is a flowchart showing each step included in the drug solution filling step. The drug solution filling step includes a positioning adjustment step (step S11), a movement step (step S12), a drug solution ejection step (step S13), an ejection finish determination step (step S14), an acceptance determination step (step S15), and a suction step (step S16).

### [Positioning Adjustment Step (Step S11)]

In the positioning adjustment step, the positioning is adjusted so that the position of the needle-like recessed portion 132 of the mold 120 placed on the XYZ stage 10 and the position of the nozzle 36 of the drug solution ejection head 34 coincide with each other. The position of the needle-like recessed portion 132 and the position of the nozzle 36 may coincide with each other so that the drug solution ejected from the nozzle 36 toward the needle-like recessed portion 132 lands on the needle-like recessed portion 132, and the positions of the two do not need to strictly coincide with each other. Here, the needle-like recessed portion 132 and the nozzle 36 are virtually positioned by detecting the position of the needle-like recessed portion 132 from the taken image.

First, the transporting jig 150 on which the mold 120 is mounted is placed on the placement surface 20A of the adsorption plate 20.

The movement controller 42 controls the XYZ stage 10 to move the mold 120 within the angle of view of the taken image of the camera 30. The imaging controller 40 controls the camera 30 to take an image of the mold 120 (an example of an imaging step). The image detector 44 calculates the position of each needle-like recessed portion 132 by analyzing the image of the mold 120 taken by the camera 30.

For example, the needle-like recessed portion 132 of the mold 120 is moved to the center within the angle of view of the taken image of the camera 30 by the XYZ stage 10, and the XY plane coordinates (X,Y) of the XYZ stage 10 at this point are detected. By performing this for all the needle-like recessed portions 132, the positions of all the needle-like recessed portions 132 can be detected.

In the image of the mold 120 taken by the camera 30, the flat portion 122 has a relatively bright brightness, and the needle-like recessed portion 132 has a relatively dark brightness. By using this contrast, the needle-like recessed portion 132 can be moved to the center within the angle of view of the taken image of the camera 30.

Instead of moving all the needle-like recessed portions 132 to the center within the angle of view of the image taken by the camera 30, only the XY plane coordinates (X,Y) of three to five needle-like recessed portions 132 may be detected and the direction (rotation) of the mold 120 in the XY plane from the coordinates and the deviation or expansion and contraction of the mold 120 in the XY plane may be analyzed to detect the positions of the other needle-like recessed portions 132.

Alternatively, the mold 120 may be provided with a plurality of alignment marks, and the XY plane coordinates (X,Y) of the needle-like recessed portion 132 may be detected by reading the alignment marks.

As described above, by detecting the position of the needle-like recessed portion 132 based on the XY plane coordinates (X,Y) of the XYZ stage 10, the needle-like recessed portion 132 and the nozzle 36 are virtually positioned. Mechanical positioning may be performed by a positioning adjustment holding device or the like.

Furthermore, the position (height) of the mold 120 in the Z direction may be adjusted by measuring the distance between the needle-like recessed portion 132 or the alignment mark and the camera 30. The distance between the nozzle 36 and the mold 120 is preferably adjusted to be 0.5 mm to 5 mm, and preferably 1 mm to 2 mm.

### [Relative Movement Step (Step S12)]

The movement controller 42 controls the XYZ stage 10 based on the detection result of the image detector 44 to move the mold 120 in the X direction and the Y direction so as to cause the position of the nozzle 36 of the drug solution ejection head 34 on the XY plane and the position of the needle-like recessed portion 132 on the XY plane to coincide with each other. That is, the position of the nozzle 36 and the position of the needle-like recessed portion 132 are caused to coincide with each other in a plan view in the direction (Z direction) parallel to the ejection direction of the drug solution from the nozzle 36.

Coordinates (X + d₁,Y + d₂) obtained by adding the distance d₁ in the X direction between the camera 30 and the nozzle 36 of the drug solution ejection head 34 and the distance d₂ in the Y direction to the coordinates (X,Y) of the needle-like recessed portion 132 calculated in step S11 are the coordinates of the nozzle 36. The movement controller 42 moves the XYZ stage 10 to the coordinates.

### [Drug Solution Ejection Step (Step S13)]

The ejection controller 46 controls the drug solution ejection head 34 to eject the drug solution from the nozzle 36. The ejected drug solution lands on the needle-like recessed portion 132. Here, one droplet of the drug solution is ejected from the nozzle 36 to one needle-like recessed portion 132 and is caused to land on the needle-like recessed portion 132. Alternatively, a plurality of droplets of the drug solution may be caused to land on one needle-like recessed portion 132.

The drug solution that lands on the needle-like recessed portion 132 needs to block the needle-like recessed portion 132, that is, be into contact with the entire circumferential surface of the wall portion of the needle-like recessed portion 132. In a case where the landed drug solution does not block the needle-like recessed portion 132, the landed drug solution cannot fill the distal end of the tapered shape of the distal end recessed portion 134 in the suction step, which will be described later. Therefore, in order to cause the position of the nozzle 36 and the position of the needle-like recessed portion 132 to coincide with each other in the relative movement step, precise positional accuracy is required. For this reason, it is necessary to precisely adjust the positioning in the positioning adjustment step.

### [Ejection Finish Determination Step (Step S14)]

The ejection controller 46 determines whether or not the drug solution has been ejected to land on all the needle-like recessed portions 132 of the mold 120. Here, the number of ejections of the drug solution ejected in the drug solution ejection step and the number of needle-like recessed portions 132 whose positions have been detected in the positioning adjustment step are compared to each other for determination.

In a case where it is determined that there is a needle-like recessed portion 132 on which the drug solution does not land, the process returns to step S12 and the same processing is performed. That is, the position on the XY plane of the needle-like recessed portion 132 to which the drug solution has not been ejected and the position on the XY plane of the nozzle 36 are caused to coincide with each other (step S12), and the drug solution is ejected from the nozzle 36 to land on the needle-like recessed portion 132 (step S13). The ejection order of the drug solution to the needle-like recessed portions 132 is not particularly limited, but from the viewpoint of shortening the total movement distance of the XYZ stage 10, it is preferable to eject the drug solution sequentially in order from the needle-like recessed portion 132 disposed at the end of the mold 120 to adjacent needle-like recessed portions 132.

In a case where it is determined that the drug solution has landed on all the needle-like recessed portions 132, the process proceeds to step S15.

### [Acceptance Determination Step (Step S15)]

In the acceptance determination step, the imaging controller 40 and the camera 30 image all the needle-like recessed portions 132 of the mold 120, and check whether or not the number of the needle-like recessed portions 132 whose openings are blocked by the drug solution is equal to or more than a predetermined reference number. This reference number is determined by the minimum number of needle-like protrusions 112 required in one percutaneous absorption sheet 100.

In a case where the number of blocked needle-like recessed portions 132 is less than the reference number, the process of this flowchart is finished as a rejected product. In a case where the number of blocked needle-like recessed portions 132 is equal to or more than the reference number, the process proceeds to step S16 as an acceptable product.

### [Suction Step (Step S16)]

The suction controller 48 drives the suction pump 22 to suction the rear surface 120B of the mold 120. By this suction, the drug solution that has landed on the needle-like recessed portion 132 fills the distal end of the tapered shape of the distal end recessed portion 134.

As above, the drug solution filling step is finished. The drug solution ejection step and the suction step may be performed at the same time. That is, the drug solution may be ejected from the nozzle 36 while suctioning is performed by the suction pump 22.

Here, the distance d₁ and the distance d₂ are treated as known values, but in a case where the distances are unknown, the distances can be obtained as follows.

A dummy mold that is not provided with the needle-like recessed portions 132 is mounted on the transporting jig 150 and placed on the placement surface 20A of the adsorption plate 20. The drug solution is ejected from the nozzle 36 to the dummy mold so as to land on the dummy mold.

Next, the XYZ stage 10 is moved in the X direction and the Y direction so that the landed drug solution is disposed at the center of the angle of view of the taken image of the camera 30. Here, the amount of movement of the XYZ stage 10 in the X direction is the distance d₁, and the amount of movement thereof in the Y direction is the distance d₂.

### <Examples>

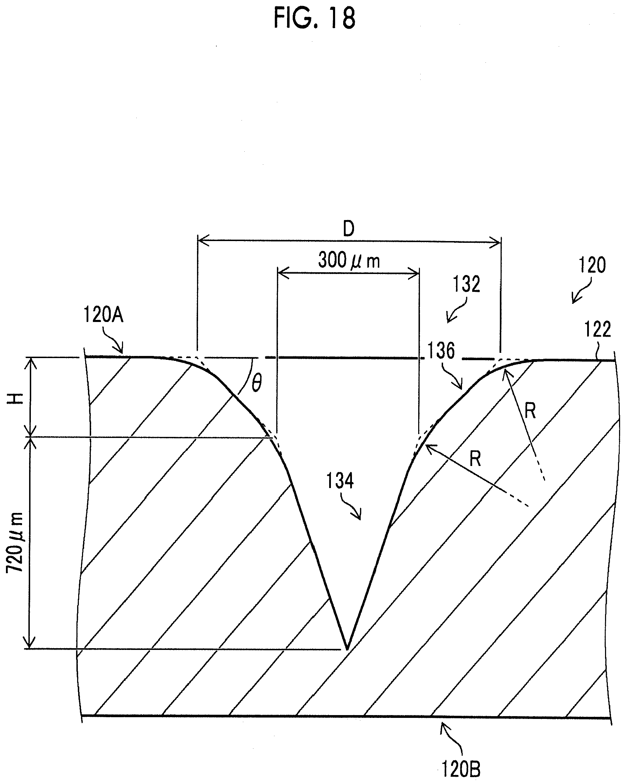

Fig. 18 is a cross-sectional view for describing the shape of the needle-like recessed portion 132 according to the example, and is a further enlarged view of Fig. 3. As illustrated in Fig. 18, the distal end recessed portion 134 has a diameter of 300 µm and a height (depth) of 720 µm. The opening diameter of the cup portion 136 (opening diameter of the needle-like recessed portion 132) is indicated as D, and the angle between the flat portion 122 of the front surface 120A of the mold 120 and the inside of the cup portion 136, which is an angle inside the mold 120, is indicated as θ. The height (depth) of the cup portion 136 is a value determined from the opening diameter D and the angle θ.

The connecting portion (an example of a first edge portion of the opening portion of the needle-like recessed portion) between the flat portion 122 of the front surface 120A and the cup portion 136 is chamfered, and the radius of curvature (first radius of curvature) of the chamfered arc is indicated as R. Due to the chamfering, the angle θ is determined as an angle formed by an extension line of the flat portion 122 of the front surface 120A and an extension line of the cup portion 136.

Furthermore, the connecting portion (an example of a second edge portion) between the cup portion 136 and the distal end recessed portion 134 is chamfered, and the radius of curvature (second radius of curvature) of the chamfered arc is indicated as R, which is the same as the first radius of curvature.

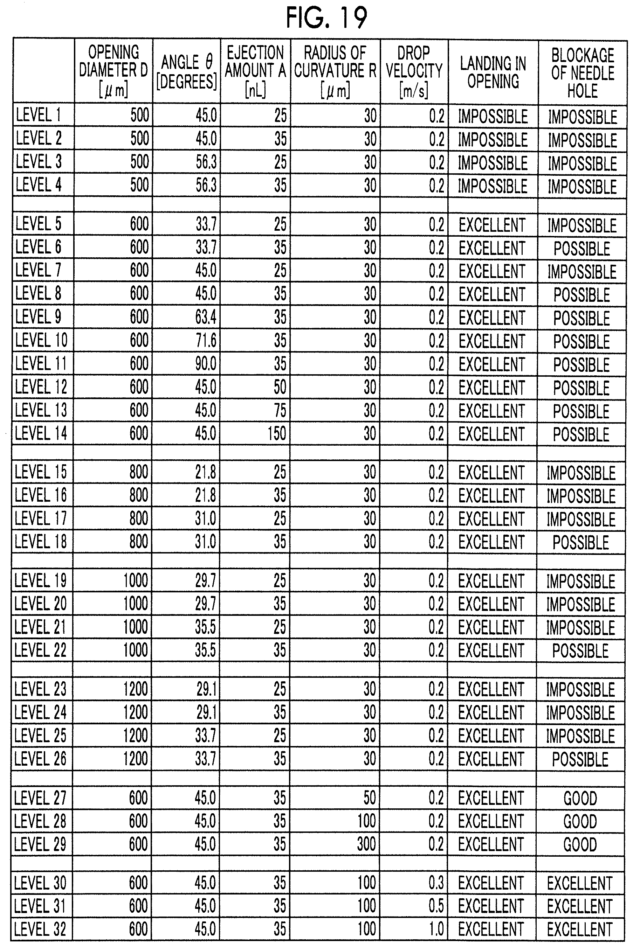

For each level with the opening diameter D, the angle θ, the radius of curvature R, the ejection amount A of the drug solution ejected from the nozzle 36, and the drop velocity V which is the flight velocity of the drug solution ejected from the nozzle 36 as parameters, the ejection (landing) of the drug solution onto the needle-like recessed portion 132 was evaluated. Fig. 19 is a table showing the parameters at each level and the evaluation results. As shown in Fig. 19, Levels 1 to 32 were evaluated.

As evaluation items, the presence or absence of landing of the drug solution in the opening and the presence or absence of blockage of the opening by the drug solution were evaluated. The criterion for evaluating the presence or absence of landing of the drug solution in the opening was determined to be successful in a case where the landing position of the ejected drug solution was inside the opening of the needle-like recessed portion 132. In addition, the criterion for evaluating the presence or absence of blockage of the opening by the drug solution is determined to be successful in a case where the drug solution landed on the needle-like recessed portion 132 was in contact with the entire circumference of the wall portion of the needle-like recessed portion 132.

As the determination criterion, a case where the success rate was 100% was determined as "excellent", a case where the success rate was 98% or more and less than 100% was determined as "good", a case where the success rate was 95% or more and less than 98% was determined as "possible", and a case where the success rate was less than 95% was determined as "impossible".

Levels 1 to 4 are levels in which the opening diameter D is 500 µm, the angle θ is 45.0 degrees or 56.3 degrees, the ejection amount A is 25 nL or 35 nL, the radius of curvature R is 30 µm, and the drop velocity V is 0.2 m/s. In Levels 1 to 4, landing in the opening was "impossible", and blockage of the opening was also "impossible" accordingly.

Levels 5 to 8 are levels in which the opening diameter D is 600 µm, the angle θ is 33.7 degrees or 45.0 degrees, the ejection amount A is 25 nL or 35 nL, the radius of curvature R is 30 µm, and the drop velocity V is 0.2 m/s.

Level 2 and Level 8 differ only in the value of the opening diameter D. In Level 2, landing in the opening was "impossible" and blockage of the opening was "impossible", whereas in Level 8, landing in the opening was "excellent" and blockage of the opening was "possible". Accordingly, it can be seen that the opening diameter D needs to be larger than 500 µm. It is considered that this is because in a case where the opening diameter D is small, it is difficult to cause the ejected drug solution to land on the needle-like recessed portion depending on the positional accuracy between the nozzle 36 and the needle-like recessed portion 132 in the relative movement step.

Levels 5 and 7 are both levels in which the ejection amount A is 25 nL. Levels 5 and 6 and Levels 7 and 8 differ only in the value of the ejection amount A. In Level 5 and Level 7, blockage of the opening was "impossible", whereas in Level 6 and Level 8, blockage of the opening was "possible". Accordingly, it can be seen that the ejection amount needs to be larger than 25 nL. It is considered that this is because in a case where the ejection amount A is small, the drug solution adheres to a part of the wall portion of the needle-like recessed portion 132, and it is difficult for the drug solution to come into contact with the entire circumference of the wall portion.

Levels 9 to 11 are levels in which the opening diameter D is 600 µm, the ejection amount A is 35 nL, the radius of curvature R is 30 µm, and the drop velocity V is 0.2 m/s, and the angles θ are 63.4 degrees, 71.6 degrees, 90.0 degrees, respectively. In addition, Level 11 had a shape in which a cylindrical portion having the opening diameter D was formed from the opening portion to a certain depth, and the cup portion 136 and the distal end recessed portion 134 were provided at the tip of the cylindrical portion because the angle θ was 90 degrees. The angle of the cup portion 136 was 45 degrees. In Levels 9 to 11, landing in the opening was "excellent" and blockage of the opening was "possible". Accordingly, it could be seen that there is no problem even if the angle θ is increased to at least 90.0 degrees. It is considered that the reason why the evaluation of the blockage of the opening does not increase even if the angle θ is increased is that since the radius of curvature R is 30 µm, the drug solution adhering to a part of the wall portion of the needle-like recessed portion 132 does not flow into the center of the needle-like recessed portion 132 and it is difficult for the drug solution to come into contact with the entire circumference of the wall portion.

Levels 12 to 14 are levels in which the opening diameter D is 600 µm, the angle θ is 45.0 degrees, the radius of curvature R is 30 µm, the drop velocity V is 0.2 m/s, and the ejection amounts A are 50 nL, 75 nL, and 150 nL, respectively. In Level 12 to Level 14, landing in the opening was "excellent" and blockage of the opening was "possible". Accordingly, it could be seen that there is no problem even if the ejection amount A is increased to at least 150 nL. It is considered that the reason why the evaluation of the blockage of the opening does not increase even if the ejection amount A is increased is that since the radius of curvature R is 30 µm, the drug solution adhering to a part of the wall portion of the needle-like recessed portion 132 does not flow into the center of the needle-like recessed portion 132 and it is difficult for the drug solution to come into contact with the entire circumference of the wall portion.

Levels 15 to 18 are levels in which the opening diameter D is 800 µm, the radius of curvature R is 30 µm, and the drop velocity V is 0.2 m/s. Levels 15 and 16 were levels in which the angle θ was 21.8 degrees, and blockage of the opening was "impossible". It is considered that this is because in a case where the angle θ is small, in a case where the landing position deviates from the center of the opening, the drug solution adhering to a part of the wall portion of the needle-like recessed portion 132 does not flow into the center of the needle-like recessed portion 132, and it is difficult for the drug solution to come into contact with the entire circumference of the wall portion.

Levels 15 and 17 were levels in which the ejection amount A was 25 nL, and blockage of the opening was "impossible". It is considered that this is because in a case where the ejection amount A is small, the drug solution adheres to a part of the wall portion of the needle-like recessed portion 132, and it is difficult for the drug solution to come into contact with the entire circumference of the wall portion.

Contrary to this, in Level 18 in which the angle θ was 31.0 degrees and the ejection amount A was 35 nL, blockage of the opening was "possible". Accordingly, it could be seen that in a case where the opening diameter D is 800 µm, the angle θ needs to be larger than 21.8 degrees and the ejection amount A needs to be larger than 25 nL.

Levels 19 to 22 are levels in which the opening diameter D is 1000 µm, the radius of curvature R is 30 µm, and the drop velocity V is 0.2 m/s. Levels 19 and 20 were levels in which the angle θ was 29.7 degrees, and blockage of the opening was "impossible". Levels 19 and 21 were levels in which the ejection amount A was 25 nL, and blockage of the opening was "impossible". Contrary to this, in Level 22 in which the angle θ was 35.5 degrees and the ejection amount A was 35 nL, blockage of the opening was "possible". Accordingly, it could be seen that in a case where the opening diameter D is 1000 µm, the angle θ needs to be larger than 29.7 degrees and the ejection amount A needs to be larger than 25 nL.

Levels 23 to 26 are levels in which the opening diameter D is 1200 µm, the radius of curvature R is 30 µm, and the drop velocity V is 0.2 m/s. Levels 23 and 24 were levels in which the angle θ was 29.1 degrees, and blockage of the opening was "impossible". Levels 23 and 25 were levels in which the ejection amount A was 25 nL, and blockage of the opening was "impossible". Contrary to this, in Level 26 in which the angle θ was 33.7 degrees and the ejection amount A was 35 nL, blockage of the opening was "possible". Accordingly, it could be seen that in a case where the opening diameter D is 1200 µm, the angle θ needs to be larger than 29.1 degrees and the ejection amount A needs to be larger than 25 nL.

Levels 27 to 29 are levels in which the opening diameter D is 600 µm, the angle θ is 45.0 degrees, the ejection amount A is 35 nL, and the drop velocity V is 0.2 m/s, and the radii of curvature R are 50 µm, 100 µmL, and 300 µm, respectively. In Levels 27 to 29, landing in the opening was "excellent" and blockage of the opening was "good". It is considered that in a case where the radius of curvature R is large and smooth, even if the landing position of the drug solution that has landed on the inside of the needle-like recessed portion 132 deviates from the center of the opening, the drug solution easily slips on the inner wall of the needle-like recessed portion 132 and the drug solution easily comes into contact with the entire circumference of the needle-like recessed portion 132. From the results of Levels 27 to 29, it could be seen that the value of the radius of curvature R was good in the range of 50 µm to 300 µm. From the results of Level 6 and the like, it could be seen that there is no problem even if the value of the radius of curvature R is 30 µm.

Levels 30 to 32 are levels in which the opening diameter D is 600 µm, the angle θ is 45.0 degrees, the ejection amount A is 35 nL, and the radius of curvature R is 100 µm, and are levels in which the drop velocities V are 0.3 m/s and 0.5 m/s, and 1.0 m/s, respectively. In Levels 30 to 32, landing in the opening was "excellent" and blockage of the opening was "excellent". It is considered that in a case where the drop velocity V is high, the ejected drug solution can be easily collected or flow into the center of the needle-like recessed portion 132. From the results of Levels 30 to 32, it could be seen that the value of the drop velocity V was good in the range of 0.3 m/s to 1.0 m/s. From the results of Level 6 and the like, it could be seen that there is no problem even if the value of the drop velocity V is 0.2 m/s.

As described above, the opening diameter D can be caused to be 600 µm or more, the angle θ can be caused to be 30.0 degrees or more, and the ejection amount A can be caused to be 30 nL or more. By causing the opening diameter to be 600 µm or more, the ejected drug solution can be easily landed on the needle-like recessed portion. In addition, by causing the angle formed with the flat portion of the mold to be 30.0 degrees or more, the landed drug solution can be easily collected or flow into the center of the needle-like recessed portion. Furthermore, by causing the ejection amount of the drug solution to be 30 nL or more, it is possible to easily block the needle-like recessed portion. It is preferable that the opening diameter D is 1200 µm or less, the angle θ is 90.0 degrees or less, and the ejection amount A is 150 nL or less.

The radius of curvature R can be caused to be 30 µm or more, and the drop velocity V can be caused to be 0.2 m/s or more. By causing the radius of curvature R to be 30 µm or more, it is possible to prevent the drug solution ejected to the needle-like recessed portion from being pinned against the first edge portion and the second edge portion, and to easily cause the drug solution to be collected or flow into the center of the needle-like recessed portion so as to fill the distal end of the needle-like recessed portion. In addition, by causing the drop velocity V to be 0.2 m/s or more, the ejected drug solution can be easily collected or flow into the center of the needle-like recessed portion. It is preferable that the radius of curvature R is 300 µm or less, and the drop velocity V is 1.0 m/s.

### <Others>

The technical scope of the present invention is not limited by the scope of the above embodiment but defined by the appended claims.

### Explanation of References

1: drug solution filling apparatus
10: XYZ stage
10A: placement surface
20: adsorption plate
20A: placement surface
21: level
22: suction pump
24: suction pipe
30: camera
32: imaging lens
34: drug solution ejection head
36: nozzle
40: imaging controller
42: movement controller
44: image detector
46: ejection controller
48: suction controller
50: electroform
52: first surface
54: protruding pattern
56: needle-like protrusion
58: second surface
70: mold
72: first mold
74: second mold
76: cavity
78: flat surface
80: adsorption plate
82: suction pipe
84: depression
86: gate
88: injection molding machine
100: percutaneous absorption sheet
100A: front surface
100B: rear surface
102: sheet portion
110: protruding pattern
112: needle-like protrusion
114: needle portion
116: frustum portion
120: mold
120A: front surface
120B: rear surface
122: flat portion
124: mold
130: recessed pattern
132: needle-like recessed portion
134: distal end recessed portion
136: cup portion
150: transporting jig
150A: placement surface
152: adsorption hole
S 1 to S5: each step of method for manufacturing percutaneous absorption sheet
S11 to S16: each step included in drug solution filling step

## Claims

1. A method for manufacturing a microneedle array, comprising:
a positioning adjustment step (s11) of adjusting positioning of a mold (70) having a plurality of needle-like recessed portions (132) on a front surface (120A), and a drug solution ejection nozzle which ejects a drug solution in a first direction;
a relative movement step (s12) of moving the mold (70) and the drug solution ejection nozzle relative to each other to cause a position of each of the plurality of needle-like recessed portions (132) and a position of the drug solution ejection nozzle to coincide with each other in a plan view in the first direction;
an ejection step (s13) of ejecting one or more droplets of the drug solution from the drug solution ejection nozzle so as to cause the drug solution to fly and land on each of the plurality of needle-like recessed portions (132); and
a suction step (s16) of suctioning a rear surface (120B) of the mold (70);
wherein each needle-like recessed portion (132) has an opening diameter of 600 µm or more, and an angle of 30.0 degrees or more with respect to a flat portion of the mold (70), and an ejection amount of the drug solution ejected to one needle-like recessed portion (132) is 30 nL or more.

2. The method for manufacturing a microneedle array according to claim 1,
wherein the opening diameter of each needle-like recessed portion (132) is 1200 µm or less.

3. The method for manufacturing a microneedle array according to claim 1 or 2,
wherein the angle of each needle-like recessed portion (132) is 90.0 degrees or less.

4. The method for manufacturing a microneedle array according to any one of claims 1 to 3,
wherein the ejection amount is 150 nL or less.

5. The method for manufacturing a microneedle array according to any one of claims 1 to 4,
wherein a first edge portion of an opening portion of each needle-like recessed portion (132) is chamfered, and
a first radius of curvature of a chamfer of the first edge portion is 30 µm or more.

6. The method for manufacturing a microneedle array according to claim 5,
wherein the first radius of curvature is 300 µm or less.

7. The method for manufacturing a microneedle array according to any one of claims 1 to 6,
wherein each needle-like recessed portion (132) comprises
a cup portion (136) provided on the front surface (120A) of the mold (70), and
a distal end recessed portion (134) which is connected to the cup portion (136) and has a tapered shape in a depth direction of the mold (70),
a second edge portion between the cup portion (136) and the distal end recessed portion (134) is chamfered, and
a second radius of curvature of a chamfer of the second edge portion is 30 µm or more.

8. The method for manufacturing a microneedle array according to claim 7,
wherein the second radius of curvature is 300 µm or less.

9. The method for manufacturing a microneedle array according to any one of claims 1 to 8,
wherein a flight velocity of the drug solution ejected from the drug solution ejection nozzle is 0.2 m/s or more.

10. The method for manufacturing a microneedle array according to claim 9, wherein the flight velocity of the drug solution ejected from the drug solution ejection nozzle is 1.0 m/s or less

11. The method for manufacturing a microneedle array according to any one of claims 1 to 10, further comprising:
an imaging step of imaging a plurality of positions of the mold (70),
wherein in the positioning adjustment step (s11), the position of each needle-like recessed portion (132) is calculated based on the plurality of positions imaged, and
in the relative movement step (s12), the position of each needle-like recessed portion (132) and a landing position of the drug solution ejected from the drug solution ejection nozzle are caused to coincident with each other based on a result of the calculation.

12. The method for manufacturing a microneedle array according to any one of claims 1 to 11,
wherein, in the suction step (s16), the rear surface (120B) of the mold (70) is suctioned after the drug solution is ejected into all the needle-like recessed portions (132) of the plurality of needle-like recessed portions (132).

13. The method for manufacturing a microneedle array according to any one of claims 1 to 12,
wherein the mold (70) has gas permeability.

14. The method for manufacturing a microneedle array according to any one of claims 1 to 13, further comprising:
a drying step of drying the drug solution with which all of the needle-like recessed portions (132) are filled.

15. The method for manufacturing a microneedle array according to any one of claims 1 to 14,
wherein the drug solution contains at least one of a drug stock solution, a sugar, or an additive.

## Patentansprüche

1. Verfahren zum Fertigen eines Mikronadelarrays, umfassend:
einen Positioniereinstellschritt (s11) des Einstellens der Positionierung einer Form (70) mit einer Mehrzahl von nadelähnlichen vertieften Abschnitten (132) auf einer Frontfläche (120A) und einer Arzneilösungs-Ausstoßdüse, die eine Arzneilösung in einer ersten Richtung ausstößt;
einen Relativbewegungsschritt (s12) des Bewegens der Form (70) und der Arzneilösungs-Ausstoßdüse relativ zueinander, um zu veranlassen, dass eine Position jedes der nadelähnlichen vertieften Abschnitte (132) und eine Position der Arzneilösungs-Ausstoßdüse miteinander in einer Draufsicht in einer ersten Richtung übereinstimmen;
einen Ausstoßschritt (s13) des Ausstoßens eines oder mehrerer Tropfen der Arzneilösung aus der Arzneilösungs-Ausstoßdüse, um zu veranlassen, dass die Arzneilösung fliegt und auf jedem der mehreren nadelähnlichen vertieften Abschnitte (132) landet; und
einen Saugschritt (s16) des Saugens an einer Rückseite (25B) der Form (70);
wobei jeder nadelähnliche vertiefte Abschnitt (132) einen Öffnungsdurchmesser von 600 µm oder mehr und einen Winkel von 30,0 Grad oder mehr bezüglich eines flachen Abschnitts der Form (70) aufweist, und eine Ausstoßmenge der von auf einem nadelähnlichen vertieften Abschnitt (132) ausgestoßenen Arzneilösung 30 nL oder mehr beträgt.

2. Verfahren nach Anspruch 1,
bei dem der Öffnungsdurchmesser jedes nadelähnlichen vertieften Abschnitts (132) 1200 µm oder weniger beträgt.

3. Verfahren nach Anspruch 1 oder 2,
bei dem der Winkel jedes nadelähnlichen vertieften Abschnitts (132) 90,0 Grad oder weniger beträgt.

4. Verfahren nach einem der Ansprüche 1 bis 3,
bei dem die Ausstoßmenge 150 nL oder weniger beträgt.

5. Verfahren nach einem der Ansprüche 1 bis 4,
bei dem ein erster Randabschnitt eines Öffnungsabschnitts jedes nadelähnlichen vertieften Abschnitts (132) abgefrast ist, und
ein erster Krümmungsradius einer Abfasung des ersten Randabschnitts 30 µm oder mehr beträgt.

6. Verfahren nach Anspruch 5,
bei dem der erste Krümmungsradius 300 µm oder weniger beträgt.

7. Verfahren nach einem der Ansprüche 1 bis 6,
bei dem jeder nadelähnliche vertiefte Abschnitt (132) aufweist:
einen Becherabschnitt (136) an der Frontseite (120A) der Form (70), und
einen distalen vertieften Endabschnitt (134), der mit dem Becherabschnitt (136) verbunden ist und in Tiefenrichtung der Form (70) eine sich verjüngende Form besitzt,
wobei ein zweiter Randabschnitt zwischen dem Becherabschnitt (136) und dem distalen vertieften Endabschnitt (134) abgefast ist, und
ein zweiter Krümmungsradius eine Abfasung des zweiten Randabschnitts 30 µm oder mehr beträgt.

8. Verfahren nach Anspruch 7,
bei dem der zweite Krümmungsradius 300 µm oder weniger beträgt.

9. Verfahren nach einem der Ansprüche 1 bis 8,
bei dem eine Fluggeschwindigkeit der aus der Arzneilösungs-Ausstoßdüse ausgestoßenen Arzneilösung 0,2 m/s oder mehr beträgt.

10. Verfahren nach Anspruch 9, bei dem die Fluggeschwindigkeit der aus der Arzneilösungs-Ausstoßdüse ausgestoßenen Arzneilösung 1,0 m/s oder weniger beträgt.

11. Verfahren nach einem der Ansprüche 1 bis 10, weiterhin umfassend:
einen Bildgebungsschritt des Abbildens einer Mehrzahl von Positionen der Form (70),
wobei in dem Positioniereinstellschritt (s11) die Position jedes nadelähnlichen vertieften Abschnitts (132) basierend auf den mehreren abgebildeten Positionen berechnet wird, und
in dem Relativbewegungsschritt (s12) die Position jedes nadelähnlichen vertieften Abschnitts (132) und eine Landungsstelle der aus der Arzneilösungs-Ausstoßdüse ausgestoßenen Arzneilösung dazu gebracht wird, basierend auf dem Berechnungsergebnis miteinander übereinzustimmen.

12. Verfahren nach einem der Ansprüche 1 bis 11,
bei dem in dem Saugschritt (s16) die Rückseite (120B) der Form (70) angesaugt wird, nachdem die Arzneilösung in sämtliche der mehreren nadelähnlichen vertieften Abschnitte (132) ausgestoßen ist.

13. Verfahren nach einem der Ansprüche 1 bis 12,
bei dem die Form (70) Gaspermeabilität besitzt.

14. Verfahren nach einem der Ansprüche 1 bis 13, weiterhin umfassend:
einen Trocknungsschritt des Trocknens der Arzneilösung, wenn sämtliche der nadelähnlichen vertieften Abschnitte (132) gefüllt sind.

15. Verfahren nach einem der Ansprüche 1 bis 14,
bei dem die Arzneilösung mindestens eine von einer Arznei-Vorratslösung, einen Zucker oder einem Additiv enthält.

## Revendications

1. Procédé de fabrication d'un réseau de microaiguilles, comprenant les étapes suivantes :
une étape d'ajustement de positionnement (s11) pour ajuster le positionnement d'un moule (70) présentant une pluralité de portions en retrait similaires à des aiguilles (132) sur une surface avant (120A), et une buse d'éjection de solution médicamenteuse, laquelle éjecte une solution médicamenteuse dans une première direction ;
une étape de déplacement relatif (s12) pour déplacer le moule (70) et la buse d'éjection de solution médicamenteuse l'un par rapport à l'autre afin de faire coïncider une position de chaque portion parmi la pluralité de portions en retrait similaires à des aiguilles (132) et une position de la buse d'éjection de solution médicamenteuse entre elles dans une vue de dessus dans la première direction ;
une étape d'éjection (s13) pour éjecter une ou plusieurs gouttelettes de la solution médicamenteuse à partir de la buse d'éjection de solution médicamenteuse de manière à faire en sorte que la solution médicamenteuse vole et atterrisse sur chaque portion de la pluralité de portions en retrait similaires à des aiguilles (132), et
une étape d'aspiration (s16) pour aspirer une surface arrière (120B) du moule (70) ;
dans lequel chaque portion en retrait similaire à des aiguilles (132) présente un diamètre d'ouverture supérieur ou égal à 600 µm, et un angle supérieur ou égal à 30,0 degrés par rapport à une portion plate du moule (70), et une quantité d'éjection de la solution médicamenteuse éjectée vers une portion en retrait similaire à des aiguilles (132) est supérieure ou égale à 30 nL.

2. Procédé de fabrication d'un réseau de microaiguilles selon la revendication 1,
dans lequel le diamètre d'ouverture de chaque portion en retrait similaire à des aiguilles (132) est inférieur ou égal à 1200 µm.

3. Procédé de fabrication d'un réseau de microaiguilles selon la revendication 1 ou 2,
dans lequel l'angle de chaque portion en retrait similaire à des aiguilles (132) est inférieur ou égal 90 degrés.

4. Procédé de fabrication d'un réseau de microaiguilles selon l'une quelconque des revendications 1 à 3,
dans lequel la quantité d'éjection est inférieure ou égale à 150 nL.

5. Procédé de fabrication d'un réseau de microaiguilles selon l'une quelconque des revendications 1 à 4,
dans lequel une première portion de bord d'une portion d'ouverture de chaque portion en retrait similaire à des aiguilles (132) est chanfreinée, et
un premier rayon de courbure d'un chanfrein de la première portion de bord est supérieur ou égal à 30 µm.

6. Procédé de fabrication d'un réseau de microaiguilles selon la revendication 5,
dans lequel le premier rayon de courbure est inférieur ou égal à 300 µm.

7. Procédé de fabrication d'un réseau de microaiguilles selon l'une quelconque des revendications 1 à 6,
dans lequel chaque portion en retrait similaire à des aiguilles (132) comprend
une portion en cuvette (136) prévue sur la surface avant (120A) du moule (70), et
une portion en retrait d'extrémité distale (134), laquelle est reliée à la portion de cuvette (136) et présente une forme à amincissement progressif dans une direction de profondeur du moule (70) ;
une seconde portion de bord entre la portion en cuvette (136) et la portion en retrait d'extrémité distale (134) est chanfreinée, et
un second rayon de courbure d'un chanfrein de la seconde portion de bord est supérieur ou égal à 30 µm.

8. Procédé de fabrication d'un réseau de microaiguilles selon la revendication 7,
dans lequel le second rayon de courbure est inférieur ou égal à 300 µm.

9. Procédé de fabrication d'un réseau de microaiguilles selon l'une quelconque des revendications 1 à 8,
dans lequel une vitesse de vol de la solution médicamenteuse éjectée à partir de la buse d'éjection de solution médicamenteuse est supérieure ou égale à 0,2 m/s.

10. Procédé de fabrication d'un réseau de microaiguilles selon la revendication 9,
dans lequel la vitesse de vol de la solution médicamenteuse éjectée à partir de la buse d'éjection de solution médicamenteuse est inférieure ou égale à 1,0 m/s.

11. Procédé de fabrication d'un réseau de microaiguilles selon l'une quelconque des revendications 1 à 10, comprenant en outre l'étape suivante :
une étape d'imagerie pour imager une pluralité de positions du moule (70),
dans lequel lors de l'étape d'ajustement de positionnement (s11), la position de chaque portion en retrait similaire à des aiguilles (132) est calculée sur la base de la pluralité de positions imagées, et
lors de l'étape de déplacement relatif (s12), il est fait en sorte que la position de chaque portion en retrait similaire à des aiguilles (132) et une position d'atterrissage de la solution médicamenteuse éjectée à partir de la buse d'éjection de solution médicamenteuse coïncident entre elles sur la base d'un résultat du calcul.

12. Procédé de fabrication d'un réseau de microaiguilles selon l'une quelconque des revendications 1 à 11,
dans lequel lors de l'étape d'aspiration (s16), la surface arrière (120B) du moule (70) est aspirée après éjection de la solution médicamenteuse dans toutes les portions en retrait similaires à des aiguilles (132) de la pluralité de portions en retrait similaires à des aiguilles (132).

13. Procédé de fabrication d'un réseau de microaiguilles selon l'une quelconque des revendications 1 à 12,
dans lequel le moule (70) présente une perméabilité aux gaz.

14. Procédé de fabrication d'un réseau de microaiguilles selon l'une quelconque des revendications 1 à 13, comprenant en outre l'étape suivante :
une étape de séchage pour sécher la solution médicamenteuse avec laquelle toutes les portions en retrait similaires à des aiguilles (132) sont remplies.

15. Procédé de fabrication d'un réseau de microaiguilles selon l'une quelconque des revendications 1 à 14,
dans lequel la solution médicamenteuse contient au moins un élément parmi une solution de base médicamenteuse, un sucre, ou un additif.
